# EUROPÄISCHE PATENTANMELDUNG

(11) **EP 1 884 222 A1**
(43) Veröffentlichungstag der Anmeldung: **06.02.2008**
(21) Anmeldenummer: 06015907.6
(22) Anmeldetag: 31.07.2006
(51) Int. Cl.: A61F 9/01, G06F 19/00

(54) **Verfahren und Einrichtung zur Fernüberwachung und Fehlerdiagnose eines ophtalmologischen Behandlungssystems**

(71) Anmelder: WaveLight AG, 91058 Erlangen (DE)
(72) Erfinder: Schwarz, Michael, 56070 Koblenz (DE); Vogler, Klaus, Dr., 90542 Eckental (DE); Kittelmann, Olaf, 14163 Berlin (DE)
(74) Vertreter: von Hellfeld, Axel

(57) **Zusammenfassung**

Die Erfindung betrifft ein automatisches Verfahren und eine automatische Einrichtung zur Fernüberwachung und Fehlerdiagnose eines ophthalmologischen Behandlungssystems basierend auf zumindest einem Betriebszustandswert zumindest einer ophthalmologischen Vorrichtung.

## Beschreibung

Die Erfindung betrifft ein automatisches Verfahren und eine automatische Einrichtung zur Fernüberwachung und Fehlerdiagnose eines ophthalmologischen Behandlungssystems.

Eines von mehreren gängigen refraktiven Verfahren der Hornhautchirurgie ist die sogenannte LASIK (Laser in sito Keratomileusis). Hierbei wird aus der Hornhaut entweder mechanisch (mittels einer oszillierenden Schneidklinge in einem sogenannten Mikrokeratom) oder optisch (mittels Laserstrahlung, z.B. sogenannten Femtosekunden-Lasersystemen) ein kleiner Deckel herausgeschnitten, der mit einem Teil seines Randes noch an der Hornhaut hängt. Anschließend wird dieser üblicherweise auch als Flap bezeichnete Deckel zur Seite geklappt, wodurch das darunter liegende Stroma zugänglich wird. Mit Laserstrahlung wird dann nach Maßgabe eines für den jeweiligen Patienten ermittelten Ablationsprofils Stromagewebe abgetragen. Der Deckel wird danach wieder zurückgeklappt, wodurch die Wunde relativ schnell verheilen kann.

Je nach Art der Bearbeitung (z.B. Inzision oder Ablation) und/oder Gewebetyp wird in der laseroptischen Augenchirurgie Laserstrahlung unterschiedlicher Wellenlängen und/oder Impulsdauern eingesetzt. Für die Anbringung von Schnitten in der Hornhaut (etwa für die Präparation eines Flaps) ist es üblich, Laserstrahlung im Bereich von etwa 350 nm oder im nahen infraroten (NIR) Wellenlängenbereich, beispielsweise zwischen 1000 und 1100 nm, mit einer Impulsdauer im Femtosekundenbereich einzusetzen. Ein derartiges System wird auch als Femtosekunden-Mikrokeratom bezeichnet. Dagegen wird für die Photoablation von Stromagewebe in der Regel auf Laserstrahlung im ultravioletten Wellenlängenbereich, beispielsweise 193 nm, zurückgegriffen, wobei die verwendete Impulsdauer auch länger sein kann, bis hin in den Nanosekundenbereich.

Eine ophthalmologische Behandlungsumgebung kann somit mehrere technisch anspruchsvolle Vorrichtungen, beispielsweise das zuvor erwähnte Femtosekunden-Mikrokeratom für den LASIK-Schnitt, ein mechanisches Mikrokeratom und das zuvor erwähnte Lasersystem im ultravioletten Wellenlängenbereich zur Photoablation, umfassen. Eine Überwachung, regelmäßige Diagnose und regelmäßige Wartung dieser technisch anspruchsvollen Vorrichtungen ist für einen Behandlungserfolg entscheidend. Jedoch kann ein Anwender dieser technisch anspruchsvollen Vorrichtungen deren Betriebszustand nicht beurteilen und folglich nicht beurteilen, wann ein Bauteil der Vorrichtung auszutauschen oder zu warten ist. Derzeit erfolgt das Bestimmen des Betriebszustandes einer ophthalmologischen Vorrichtung durch Auswerten umfangreicher Listen mit Betriebszustandswerten, die von der ophthalmologischen Vorrichtung während des Betriebes angelegt werden. Derartige Listen umfassen beispielsweise den Strom einer Pumpdiode, die Impulsanzahl, die Patientenzahl, die Betriebsdauer, die Impulsdauer, die Energieeinstellungen und dergleichen. Dazu muss ein Servicetechniker vor Ort in der ophthalmologischen Behandlungsumgebung erscheinen und die Listen mit Betriebszustandswerten manuell auswerten. Eine manuelle Auswertung stellt immer eine subjektive Entscheidung dar, ferner ist ein Servicetechniker nur begrenzt in der Lage, den Betriebszustand einer ophthalmologischen Vorrichtung zu beurteilen, wenn dafür mehrere miteinander zusammenhängende Betriebszustandswerte beurteilt werden müssen. Ferner kann während der Auswertung der Listen mit Betriebszustandswerten die ophthalmologische Vorrichtung nicht zur Behandlung eines Patienten verwendet werden. Zudem sind die ophthalmologischen Vorrichtungen über die Welt verteilt, so dass das regelmäßige Erscheinen eines Servicetechnikers sowohl für den Hersteller als auch für den Anwender der ophthalmologischen Vorrichtung aufwändig ist. Das Festlegen eines festen Austauschzeitpunktes eines verschleißbehafteten Bauteils einer ophthalmologischen Vorrichtung ist ungeeignet, da das Bauteil unter Umständen vorzeitig verschlissen sein kann, so dass der Behandlungserfolg und/oder der Patient gefährdet wird, oder das Bauteil unter Umständen noch einsatzfähig sein kann, wodurch die Betriebskosten unnötig erhöht werden.

Die US 2001/0020195 A1 beschreibt ein System zum Überwachen einer Mehrzahl von gleichen Lithographielaservorrichtungen in einer Fabrik zur Herstellung von integrierten Schaltungen. Jeder Laservorrichtung in der Fabrik ist ein Terminalserver zugeordnet. Jede Fabrik umfasst einen zentralen Kontrollserver, der Daten von dem Terminalserver der einzelnen Laservorrichtungen der Fabrik sammelt und eine zusammengefasste Information daraus in Form einer Webseite zugangsberechtigten Personen zugänglich macht. Das Personal des Herstellers einer derartigen Laservorrichtung kann durch Auswerten der Informationen auf der durch den zentralen Kontrollserver erzeugten Webseite bestimmen, wann eine Laservorrichtung gewartet werden muss oder ein Bauteil ausgetauscht werden muss.

Die EP 1 150 401 A2 offenbart eine Laserüberwachungsvorrichtung für die Halbleiterfertigung, die Zustandsdaten von einer Laservorrichtung abruft, sobald ein Ereignis auftritt. Dadurch wird eine Datenbank in der Laserüberwachungsvorrichtung aktualisiert, und es werden Ausgabedaten erzeugt, die auf einer Anzeigeeinrichtung angezeigt werden. Die Zustandsdaten umfassen Daten, die den Betriebszustand anzeigen, Qualitätsdaten, Daten, die den Betriebszustand der gewarteten Bauteile anzeigen, und eine Fehlerhistorie. Die Laserüberwachungsvorrichtung kann auf einer Anzeigeeinrichtung, die in der Nähe der Laservorrichtung angeordnet ist, Trends über die Zeit anzeigen, so dass ein Servicetechniker vorhersagen kann, wann ein Bauteil der Laservorrichtung auszutauschen ist. Die Daten können auch mit voreingestellten Beurteilungskriterien verglichen werden, um festzustellen, ob sich ein Bauteil der Laservorrichtung in der Nähe des Endes der Lebensdauer befindet. Die Laserüberwachungsvorrichtung kann auch die Lebensdauer eines Bauteils und etwaige Probleme vorhersagen. Es kann eine Mehrzahl von Laservorrichtungen an die Laserüberwachungsvorrichtung angeschlossen sein.

Die US 6,749,566 B2 zeigt eine Patientenüberwachungseinrichtung, die an einem Patienten angeordnet wird und mit mehreren am Patienten angeordneten Sensoren verbunden ist. Die Patientenüberwachungseinrichtung sammelt Daten von den Sensoren und sendet diese an eine übergeordnete Patientenüberwachungsverarbeitungseinrichtung.

Es ist eine Aufgabe der Erfindung, einen Wartungszeitpunkt eines Bauteils einer ophthalmologischen Vorrichtung zu bestimmen.

Die Aufgabe wird durch ein Verfahren zum Bestimmen eines Wartungszeitpunktes für zumindest ein Bauteil einer ophthalmologischen Vorrichtung gelöst. Das Verfahren ermittelt zumindest einen Betriebszustandswert, der auf den Betriebszustand zumindest eines Bauteils der ophthalmologischen Vorrichtung hinweist. Der zumindest eine Betriebszustandswert wird von der zumindest einen ophthalmologischen Vorrichtung an eine entfernte Überwachungseinrichtung übertragen. Der Wartungszeitpunkt des zumindest einen Bauteils der zumindest einen ophthalmologischen Vorrichtung wird basierend auf dem zumindest einen Betriebszustandswert der zumindest einen ophthalmologischen Vorrichtung in der entfernten Überwachungseinrichtung bestimmt.

Die ophthalmologische Vorrichtung kann sich in einer Behandlungsumgebung, beispielsweise einer Praxis oder einem Krankenhaus, und die Überwachungseinrichtung kann sich entfernt davon, beispielsweise beim Hersteller und/oder einer Wartungs-Organisationseinheit, befinden.

Die ophthalmologische Vorrichtung kann beispielsweise ein refraktives Lasersystem oder ein Femtosekunden-Mikrokeratomsystem sein. Der Betriebszustandswert kann beispielsweise die Impulsanzahl, die Impulsdauer, die Impulsenergie, die maximale Temperatur des Lasersystems, die durchschnittliche Temperatur des Lasersystems, die Betriebsspannung, die Betriebsstunden (seit der letzten Wartung) oder dergleichen sein. Der Wartungszeitpunkt eines Bauteils einer Laservorrichtung ist beispielsweise der Zeitpunkt, zu dem die technische Funktion des entsprechenden Bauteils, beispielsweise zum Erreichen eines Behandlungserfolges, nicht mehr gewährleistet ist, weil das entsprechende Bauteil einen vorgegebenen technischen Spezifikationsbereich nicht mehr einhält. Das kann dann der Fall sein, wenn ein Betriebszustandswert, beispielsweise die maximal zulässige Impulsanzahl, die Energiewerte und/oder der Spannungsbereich, den Sollbereich verlässt. Der Wartungszeitpunkt zumindest eines Bauteils der ophthalmologischen Vorrichtung kann der Zeitpunkt sein, zu dem ein Bauteil ausgetauscht werden muss, eine Justage oder eine erneute Einstellung durchgeführt werden muss, Hilfsstoffe ausgetauscht oder aufgefüllt werden müssen oder dergleichen.

Der Ausdruck Bauteil umfasst sowohl ein einfaches Element als auch eine komplexe Komponente oder Einheit einer ophthalmologischen Vorrichtung. Das Bestimmen kann ein Berechnen, eine Prognose und/oder ein Schätzen umfassen.

Das Verfahren kann ferner den zumindest einen Betriebszustandswertes bei einer Mehrzahl von ophthalmologischen Vorrichtungen ermitteln. Der zumindest eine Betriebszustandswert wird von der Mehrzahl von ophthalmologischen Vorrichtungen an die entfernte Überwachungseinrichtung übertragen. Der Wartungszeitpunkt des zumindest einen Bauteils zumindest einer der Mehrzahl von ophthalmologischen Vorrichtungen wird basierend auf dem zumindest einen Betriebszustandswert der Mehrzahl von ophthalmologischen Vorrichtungen in der entfernten Überwachungseinrichtung bestimmt.

Das Überwachungsverfahren hat den Vorteil, dass es von einer menschlichen Entscheidung unabhängig ist. Da das Überwachungssystem mehrere ophthalmologische Vorrichtungen überwacht, können die Ergebnisse aus der Auswertung der Betriebszustandswerte zentral und schnell einbezogen werden, beispielsweise über adaptive Entscheidungsalgorithmen. Da das Überwachungsverfahren Betriebszustandswerte mehrerer ophthalmologischer Vorrichtungen berücksichtigt, können genauere Vorhersagen über die Lebensdauer und/oder den Austauschzeitpunkt von Bauteilen der ophthalmologischen Vorrichtungen bereitgestellt werden. Durch das Überwachungsverfahren können das Betriebsverhalten, der Verschleißverlauf und/oder die Verschleißerscheinungen eines Typs von ophthalmologischen Vorrichtungen bestimmt werden. Es können auch Bauteile erkannt werden, die verglichen mit dem gleichen Bauteil einer anderen ophthalmologischen Vorrichtung ungewöhnlich schnell einer Verschlechterung oder einem Verschleiß unterliegen, so dass beispielsweise ein Servicetechniker informiert werden kann, dieses Bauteil möglichst bald zu untersuchen, zu prüfen und/oder auszutauschen. Aufgrund des zumindest einen Betriebszustandwertes können die Laservorrichtung und/oder deren Bauteile aus der Ferne überwacht werden und etwaige Fehler können aus der Ferne diagnostiziert werden. Insbesondere kann ein Betriebszustandswert eines Bauteils einer ophthalmologischen Vorrichtung mit dem Betriebszustandswert eines Bauteils einer anderen ophthalmologischen Vorrichtung automatisch verglichen werden, um den Wartungszeitpunkt zu bestimmen.

Jede ophthalmologische Vorrichtung kann unabhängig von einer anderen ophthalmologischen Vorrichtung Daten zu einem geeigneten Zeitpunkt übertragen. Es ist nicht erforderlich, dass alle ophthalmologischen Vorrichtungen Daten zum gleichen Zeitpunkt übertragen.

Der Schritt des Bestimmens des Wartungszeitpunktes des zumindest einen Bauteils der zumindest einen ophthalmologischen Vorrichtung kann basierend auf einer Mehrzahl von Betriebszustandswerten der zumindest einen ophthalmologischen Vorrichtung durchgeführt werden. Werden mehrere unterschiedliche Betriebszustandswerte einer ophthalmologischen Vorrichtung kombiniert, kann der Wartungszeitpunkt genauer bestimmt werden. Beispielsweise können bei einer Laservorrichtung die Impulsanzahl, die Impulsenergie und/oder Impulsdauer kombiniert werden, um den Betriebszustand der Laservorrichtung zu bestimmen.

Der Schritt des Bestimmens des Wartungszeitpunktes des zumindest einen Bauteils der zumindest einen ophthalmologischen Vorrichtung kann basierend auf einer Änderung des zumindest einen Betriebszustandswertes durchgeführt werden. Der Schritt des Bestimmens des Wartungszeitpunktes des zumindest einen Bauteils der zumindest einen ophthalmologischen Vorrichtung kann basierend auf einer zeitlichen Änderung und/oder auf einer von der Einsatzhäufigkeit abhängigen Änderung eines Betriebszustandswertes der ophthalmologischen Vorrichtung durchgeführt werden. Durch die Berücksichtigung der Änderung eines Betriebszustandswertes kann eine genauere Bestimmung des Wartungszeitpunktes des Bauteils der ophthalmologischen Vorrichtung bereitgestellt werden. Die Einsatzhäufigkeit kann beispielsweise durch die Anzahl der von einer Laservorrichtung abgegebenen Impulse bestimmt werden.

Insbesondere eine verglichen mit gleichen Bauteilen einer anderen ophthalmologischen Vorrichtung unübliche Änderung, beispielsweise eine sprunghafte Änderung, kann erkannt werden und ein Servicetechniker wird automatisch informiert und in die Lage versetzt, das entsprechende Bauteil frühzeitig zu untersuchen und/oder auszutauschen. Der Schritt des Bestimmens des Wartungszeitpunktes des zumindest einen Bauteils der zumindest einen ophthalmologischen Vorrichtung kann unter Verwendung einer Extrapolationsfunktion durchgeführt werden. Die Extrapolationsfunktion kann basierend auf der Änderung des zumindest einen Betriebszustandswertes einer ophthalmologischen Vorrichtung bestimmt werden. Weist beispielsweise ein Bauteil einer einzelnen ophthalmologischen Vorrichtung eine unübliche Änderung auf, kann eine Extrapolationsfunktion nur für dieses Bauteil dieser ophthalmologischen Vorrichtung bereitgestellt werden.

Ferner kann die Extrapolationsfunktion basierend auf der Änderung des zumindest einen Betriebszustandswertes einer Mehrzahl von ophthalmologischen Vorrichtungen bestimmt werden. Dadurch wird eine von Exemplarstreuungen unabhängige Extrapolationsfunktion erhalten. Beispielsweise kann bei der Bestimmung des Wartungszeitpunktes des Bauteils einer ophthalmologischen Vorrichtung zuerst die Extrapolationsfunktion verwendet werden, die basierend auf einer Mehrzahl von ophthalmologischen Vorrichtungen bestimmt wurde, und falls das entsprechende Bauteil eine unübliche Änderung aufweist, kann der Wartungszeitpunkt mit der Extrapolationsfunktion bestimmt werden, die auf der Änderung des zumindest einen Betriebszustandswertes lediglich einer ophthalmologischen Einrichtung basiert.

Dem Fachmann sind mehrere Verfahren zum Bestimmen der Extrapolationsfunktion bekannt, beispielsweise kann die Extrapolationsfunktion durch Interpolation der über die Einsatzdauer ermittelten Betriebszustandswerte bestimmt werden.

Der Wartungszeitpunkt des zumindest einen Bauteils der zumindest einen ophthalmologischen Vorrichtung kann unter Verwendung eines statistischen Verfahrens bestimmt werden. Der Wartungszeitpunkt des zumindest einen Bauteils der zumindest einen ophthalmologischen Vorrichtung kann auch durch einen atypischen Wert des Betriebszustandswertes oder durch eine atypische Änderung des Betriebszustandswertes bestimmt werden.

Der Schritt des Übertragens des zumindest einen Betriebszustandswertes kann ein Senden des zumindest einen Betriebszustandswertes an die entfernte Überwachungseinrichtung umfassen. Das Senden kann aufgrund eines vorbestimmten Ereignisses in zumindest einer ophthalmologischen Vorrichtung und/oder durch manuelle Auslösung erfolgen. Da die ophthalmologische Vorrichtung die Daten an die entfernte Überwachungseinrichtung sendet, kann sichergestellt werden, dass die Daten nur gesendet werden, wenn die ophthalmologische Vorrichtung nicht für eine Operation am Auge eingesetzt wird. Vorzugsweise erfolgt das Senden des zumindest einen Betriebszustandswertes beim Einschalten und/oder Hochlauf der ophthalmologischen Vorrichtung.

Das Verfahren kann ferner den zumindest einen Betriebszustandswert an eine Sammeleinrichtung übertragen, die dazu ausgebildet ist, zumindest einen Betriebszustandswert einer Mehrzahl von ophthalmologischen Vorrichtungen zu empfangen, und den zumindest einen Betriebszustandswert von der Sammeleinrichtung an die entfernte Überwachungseinrichtung zu senden. Ferner können Betriebszustandswerte von einer Mehrzahl von ophthalmologischen Vorrichtungen unterschiedlichen Typs an die Sammeleinrichtung übertragen werden. Die Sammeleinrichtung empfängt Betriebszustandswerte von einer Mehrzahl von ophthalmologischen Vorrichtungen, die auch von einem unterschiedlichen Typ sein können, speichert diese Werte und sendet diese Werte zu einem geeigneten Zeitpunkt an die entfernte Überwachungseinrichtung. Die Mehrzahl von ophthalmologischen Vorrichtungen kann sich beispielsweise in einem Behandlungsbereich befinden. Die Sammeleinrichtung hat den Vorteil, dass nicht jede ophthalmologische Vorrichtung eine Kommunikationsschnittstelle mit der Überwachungseinrichtung benötigt. Die Sammeleinrichtung kann auch derart ausgebildet sein, dass sie ein lokales Netzwerk mit zumindest einer ophthalmologischen Vorrichtung von einem anderen Netzwerk, beispielsweise dem Internet, trennt. Das Trennen kann eine Funktionalität einer Vermittlungseinrichtung, einer Firewall und/oder eines Routers umfassen. Die Anmelderin behält sich ausdrücklich vor, auf die Sammeleinrichtung separat Schutz zu beanspruchen.

Die entfernte Überwachungseinrichtung kann aus dem zumindest einen Betriebszustandswert einen Vorgabewert berechnen und diesen an zumindest eine der Mehrzahl von ophthalmologischen Vorrichtungen senden. Der Vorgabewert kann beispielsweise die Hochspannungseinstellung des Laserkopfes betreffen. Das Verfahren kann derart ausgebildet sein, dass die Änderung von dem Anwender oder von dem Servicetechniker bestätigt werden muss, bevor die ophthalmologische Vorrichtung den geänderten Vorgabewert im Betrieb verwendet.

Die Erfindung betrifft ferner ein Computerprogrammprodukt mit einem auf einem maschinenlesbaren Träger gespeicherten Programmcode zur Durchführung des zuvor beschriebenen Verfahrens und seiner Weiterbildungen, wenn der Programmcode in einem Computer geladen ist oder in einem Computer ausgeführt wird. Das Computerprogrammprodukt kann beispielsweise eine Diskette, CD, DVD oder dergleichen sein. Unter Computer wird jegliche Vorrichtung verstanden, die Aktionen in Abhängigkeit von einem erhaltenen Programmcode ausführen kann. Es versteht sich, dass die zuvor beschriebenen Schritte auf einer beliebigen geeigneten Vorrichtung automatisch ausgeführt werden.

Die Erfindung betrifft ferner eine Überwachungseinrichtung, die dazu ausgelegt ist, mit zumindest einer ophthalmologischen Vorrichtung zusammenzuwirken, die dazu ausgebildet ist, zumindest einen Betriebszustandswert zumindest eines Bauteils zu ermitteln und zu senden. Die Überwachungseinrichtung umfasst eine Empfangseinrichtung, die dazu ausgebildet ist, den zumindest einen Betriebszustandswert von der zumindest einen ophthalmologischen Vorrichtung zu empfangen, und eine Auswerteeinrichtung, die dazu ausgebildet ist, einen Wartungszeitpunkt für zumindest ein Bauteil der zumindest einen ophthalmologischen Vorrichtung basierend auf dem zumindest einen Betriebszustandswert der zumindest einen ophthalmologischen Vorrichtung zu bestimmen.

Die Überwachungseinrichtung kann durch ein Computersystem mit einem Allzweck- oder Spezialzweckprozessor, einem Speicher und einer Kommunikationsschnittstelle aufgebaut sein. Die Überwachungseinrichtung kann auch aus speziell ausgebildeten Hardwarekomponenten, beispielsweise anwendungsspezifischen Schaltkreisen (ASIC), FPGAs oder dergleichen aufgebaut sein.

Die Auswerteeinrichtung kann dazu ausgebildet sein, den Wartungszeitpunkt für zumindest ein Bauteil der zumindest einen ophthalmologischen Vorrichtung basierend auf dem zumindest einen Betriebszustandswert einer Mehrzahl von ophthalmologischen Vorrichtungen zu bestimmen.

Die Auswerteeinrichtung kann dazu ausgebildet sein, die zuvor genannten Weiterbildungen des Verfahrens zum Bestimmen des Wartungszeitpunktes des zumindest einen Bauteils der zumindest einen ophthalmologischen Vorrichtung auszuführen. Ferner kann die Überwachungseinrichtung eine Extrapolationseinrichtung umfassen, die dazu ausgebildet ist, die zuvor bezüglich der Extrapolationsfunktion beschriebenen Weiterbildungen des Verfahrens auszuführen.

Die Erfindung betrifft ferner ein Überwachungssystem, das die zuvor beschriebene Überwachungseinrichtung und eine Sammeleinrichtung umfasst, die dazu ausgebildet ist, zumindest einen Betriebszustandswert einer Mehrzahl von ophthalmologischen Vorrichtungen zu empfangen und den zumindest einen Betriebszustandswert zu der entfernten Einrichtung zu senden. Die Sammeleinrichtung kann dazu ausgebildet sein, zumindest einen Betriebszustandswert einer Mehrzahl von ophthalmologischen Vorrichtungen unterschiedlichen Typs zu empfangen, zu speichern und an die entfernte Einrichtung zu senden. Befinden sich in einem ophthalmologischen Behandlungsbereich mehrere ophthalmologische Vorrichtungen unterschiedlichen Typs, kann die Sammeleinrichtung Betriebszustandswerte einer jeden ophthalmologischen Vorrichtung sammeln und zu einem geeigneten Zeitpunkt an die Überwachungseinrichtung weiterleiten. Somit benötigt nicht jede ophthalmologische Vorrichtung eine eigene Kommunikationsschnittstelle mit der Überwachungseinrichtung.

Mit der von der ophthalmologischen Vorrichtung entfernten Überwachungseinrichtung kann die Funktion der ophthalmologischen Vorrichtung geprüft werden. Durch Analyse eines Trendes von Betriebszustandswerten können Verschlechterungen erkannt werden. Somit kann ein Wartungszeitpunkt prognostiziert werden. Die Wartung kann folglich vor dem Ausfall eines Bauteils erfolgen.

Die Erfindung wird nachfolgend unter Bezugnahme auf die begleitenden Zeichnungen detaillierter beschrieben. Es zeigen:
Fig. 1 eine schematische Darstellung einer Mehrzahl von ophthalmologischen Vorrichtungen, die über ein Netzwerk mit einer Überwachungseinrichtung verbunden sind;
Fig. 2 eine weitere Ausführungsform einer Sammeleinrichtung;
Fig. 3 einen Graph, der die zum Erreichen der Sollenergie eines Laserimpulses erforderliche Hochspannung in Abhängigkeit von der akkumulierten Impulsanzahl oder Lebensdauer darstellt; und
Fig. 4 einen Graph der Schwankung der Laserimpulsenergie bei einem refraktiven Lasersystem über die Impulsanzahl oder die Betriebsstunden.

Ein ophthalmologischer Behandlungsbereich mit einer ersten Laservorrichtung 12 und einer zweiten Laservorrichtung 22, eine dritte Laservorrichtung 32 und eine vierte Laservorrichtung 42 sind mit einer Überwachungseinrichtung 2 verbunden. Die ophthalmologische Behandlungsumgebung, die dritte Laservorrichtung 32 und die vierte Laservorrichtung 42 sowie die Überwachungseinrichtung 2 können räumlich voneinander getrennt sein und sich an unterschiedlichen Orten der Welt befinden. Die erste Laservorrichtung 12 und die zweite Laservorrichtung 22 kommunizieren über eine Kommunikationseinrichtung 14 bzw. 24, über ein lokales Netzwerk 10, über eine Kommunikationseinrichtung 20 des ophthalmologischen Behandlungsbereichs und über das Internet 1 mit der Kommunikationseinrichtung 3 der Überwachungseinrichtung 2. Die Kommunikationseinrichtung 20 des ophthalmologischen Behandlungsbereichs und/oder die Kommunikationseinrichtung 3 der Überwachungseinrichtung 2 können eine so genannte Firewall umfassen. Die dritte Laservorrichtung 32 ist über ein Modem 34, eine Telefonleitung 38, einen nicht gezeigten Internetdienstanbieter und das Internet 1 mit der Kommunikationseinrichtung 3 der Überwachungseinrichtung 2 verbunden. Die vierte Laservorrichtung 42 ist über eine Kommunikationseinrichtung 44, die eine ISDN-Schnittstelle, eine Telefonleitungsschnittstelle oder ein Modem sein kann, über eine Telefonleitung 48 mit der Kommunikationseinrichtung 3 der Überwachungseinrichtung 2 verbunden.

Jede Laservorrichtung 12, 22, 32, 42 umfasst einen Strahlengang 16, 26, 36, 46, der die Laserstrahlung zu dem zu behandelnden Auge führt. Die erste Laservorrichtung 12 kann beispielsweise eine Femtosekunden-Laservorrichtung sein, die Laserstrahlung im nahen infraroten Wellenlängenbereich bereitstellt, um einen Schnitt in der Hornhaut eines Auges zu erzeugen. Die zweite Laservorrichtung 22 kann Laserstrahlung im ultravioletten Wellenlängenbereich zur Ablation von Stromagewebe bereitstellen, um das Stromagewebe neu zu formen.

Während des Betriebes ermittelt jede Laservorrichtung eine Mehrzahl von Betriebszustandswerten. Eine Femtosekunden-Laservorrichtung erzeugt beispielsweise die folgenden Betriebszustandswerte: einen Strom der Pumpdiode, eine Impulsanzahl, eine Patientenanzahl, eine Betriebsdauer, eine Impulsdauer, eine Femtosekundenlaser-Impulsenergie, Energieeinstellungen, eine Strahllagestabilität, Informationen über ein Strahlprofil, eine Qualität des Strahlprofils, Informationen über eine Wellenfront, eine Fokuslage sowie Fokusstabilität, mechanische Einstellungen, Betriebsstunden, Anzahl der Operationen, eine Geräte- und Umgebungstemperatur, eine Hochlaufzeit (warm-up time), eine Nutzeridentifizierung, sowie Warnungen und Fehlermeldungen. Zusätzlich können folgende Betriebszustandswerte bereitgestellt werden: Druck einer Vorvakuum-Pumpe für einen Saugring, Dauer der LASIK-Schnitte und/oder eine Statistik der verwendeten Parameter für Femtosekunden-Mikrokeratomschnitte. Eine Excimer-basierte, refraktive Laservorrichtung zur Ablation von Stromagewebe ermittelt beispielsweise die folgenden Betriebszustandswerte: eine Impulsanzahl (beispielsweise seit der letzten Wartung), eine Betriebsdauer, eine Patientenzahl, Anzahl der Augenoperationen, einen Gasdruck in einer Laserröhre, eine Hochspannungseinstellung eines Laserkopfes, eine Impulsenergie, eine Gerätetemperatur, Warn- oder Fehlermeldungen, Werte über den Betrieb einer Patientenliege, eine Anzahl von Gaswechseln, Anzahl der Frischgasfüllungen, Füllzustand der Gasflaschen (Druck), ein Alter eines Fluorfilters und Betriebsstunden des Fluorfilters. Ferner können Betriebszustandswerte über die Scanner-Funktionalität und die Eyetracker-Funktionalität bereitgestellt werden. Wie zuvor erwähnt, kann die Überwachungseinrichtung auch mit anderen ophthalmologischen Einrichtungen, beispielsweise einem mechanischen Mikrokeratom, intelligenten optischen Komponenten, Aktoren, Messgeräten und dergleichen, verbunden sein und zumindest einen Betriebszustandswert von diesen empfangen. Die Überwachungseinrichtung und das Verfahren zum Bestimmen des Wartungszeitpunktes des zumindest einen Bauteiles der zumindest einen ophthalmologischen Vorrichtung können einen Betriebszustandswert von unterschiedlichen ophthalmologischen Vorrichtungen empfangen, erhalten und/oder verarbeiten.

Die zuvor erwähnten Betriebszustandswerte können während des Betriebes ständig aktualisiert werden und in einer (nicht gezeigten) Speichereinrichtung jeder Laservorrichtung gespeichert werden. Die ermittelten Betriebszustandswerte werden, wie zuvor beschrieben, an die Überwachungseinrichtung 2 gesendet. Das Senden kann während des Einschaltens der Laservorrichtung, aufgrund eines Ereignisses, beispielsweise eines Fehlerereignisses, und/oder auf Bedieneranforderung erfolgen.

Die Laservorrichtung stellt dabei sicher, dass das Senden nur erfolgt, wenn die Laservorrichtung nicht zur Behandlung eines Patienten eingesetzt wird. Die dabei anfallende Datenmenge kann zwischen etwa einem Kilobyte und etwa vier Megabyte betragen. Je nach Bandbreite der Kommunikationsverbindung ergeben sich dabei Übertragungszeiten zwischen einigen Sekunden (DSL, Kabelmodem) und etwa 10 Minuten (Modem für eine analoge Telefonleitung).

Die Überwachungseinrichtung 2 umfasst die Kommunikationseinrichtung 3 mit einer fakultativen Firewall-Funktionalität, eine Auswerteeinrichtung 4 und eine Extrapolationseinrichtung 5. Ferner kann die Überwachungseinrichtung 2 eine Datenbank 6 zum Speichern der erhaltenen Betriebszustandswerte umfassen. An der Überwachungseinrichtung 2 kann eine Anzeigeeinrichtung 7 angeschlossen sein, wobei die Anzeigeeinrichtung 7 integral mit der Überwachungseinrichtung 2 ausgebildet sein kann. Die Kommunikationseinrichtung 3, die Auswerteeinrichtung 4, die Extrapolationseinrichtung 5, die Datenbank 6 und die Anzeigeeinrichtung 7 sind über eine Kommunikationsverbindung 8 miteinander verbunden, die als lokales Netzwerk, Bus und/oder als Interprogrammkommunikation bzw. Funktionsaufruf innerhalb des gleichen Programms ausgebildet sein kann. Die erhaltenen Betriebszustandswerte können in der Datenbank 6 gespeichert werden.

Die Auswerteeinrichtung 4 wertet die erhaltenen Betriebszustandswerte der Mehrzahl von Laservorrichtungen 12, 22, 32, 42 aus. Sind die Laservorrichtungen 12, 32 und 42 vom gleichen Typ, kann beispielsweise deren abgegebene Impulsenergie bei gleichen Einstellungen der Laservorrichtung verglichen werden, so dass Bauteile mit einer niedrigen Leistungsfähigkeit und/oder verschlechterten Funktionsfähigkeit erkannt werden können. Die Auswerteeinrichtung 4 kann statistische Verfahren, beispielsweise das Bilden eines Mittelwertes und/oder einer Standardabweichung, verwenden, um die Betriebszustandswerte auszuwerten. Weicht beispielsweise ein Betriebszustandswert einer Laservorrichtung um ein Mehrfaches einer Standardabweichung vom Mittelwert des gleichen Betriebszustandswertes der anderen Laservorrichtungen des gleichen Typs ab, kann die Auswerteeinrichtung 4 auf der Anzeigeeinrichtung 7 eine Warnung ausgeben. Der Betriebszustandswert kann sich lediglich auf ein Bauteil der Laservorrichtung beziehen, was ermöglicht, dass gleiche oder ähnliche Bauteile von Laservorrichtungen unabhängig vom Typ der Laservorrichtung mit dem statistischen Verfahren überwacht werden können.

Bei einer weiteren Ausführungsform berücksichtigt die Auswerteeinrichtung mehrere Betriebszustandswerte in Kombination bzw. gleichzeitig, um den Betriebszustand eines Bauteils der Laservorrichtung zu ermitteln. Beispielsweise berücksichtigt die Auswerteeinrichtung 4 die Fokuslage und die Impulsenergie des von einer Lichterzeugungseinrichtung der Laservorrichtung abgegebenen Lichtstrahls, um den Zustand der Lichterzeugungseinrichtung und deren weitere Lebensdauer zu bestimmen. In Abhängigkeit von der bestimmten weiteren möglichen Einsatzdauer oder der noch verbleibenden Lebensdauer des Bauteils kann eine Warnung und/oder ein Austauschzeitpunkt an der Anzeigeeinrichtung ausgegeben werden. Da Betriebszustandswerte mehrerer Laservorrichtungen miteinander verglichen werden, lassen sich Bauteile, die eine verschlechterte technische Funktion aufweisen, einfach bestimmen. Ferner wird die Patientensicherheit durch das frühzeitige Erkennen eines Bauteils mit einem unüblichen Verhalten erhöht.

Die Auswerteeinrichtung bestimmt bei einer bevorzugten Ausführungsform den Wartungszeitpunkt, beispielsweise den Austauschzeitpunkt, eines Bauteils der Laservorrichtung aufgrund einer Änderung des zumindest einen Betriebszustandswertes. Dabei wird die Änderung des zumindest einen Betriebszustandswertes basierend auf einer zeitlichen Änderung und/oder einer Änderung in Abhängigkeit von der Einsatzhäufigkeit der Laservorrichtung bestimmt. Die Änderung eines Betriebszustandswertes kann über die Betriebszeit der Laservorrichtung, über die von der Laservorrichtung abgegebenen Impulse und/oder über die von der Laservorrichtung abgegebene Laserenergie betrachtet werden. Die Betrachtung der Änderung eines Betriebszustandswertes ermöglicht eine genauere Bestimmung des Wartungszeitpunktes eines Bauteiles der Laservorrichtung.

Eine Extrapolationseinrichtung 5, die mit der Auswerteeinrichtung 4 auch integral ausgebildet sein kann, bestimmt den Wartungszeitpunkt eines Bauteils einer Laservorrichtung mittels einer Extrapolationsfunktion, bei der zumindest ein Betriebszustandswert einbezogen wird. Die Extrapolationsfunktion kann beispielsweise durch Interpolieren der bisher ermittelten Betriebszustandswerte und einem anschließenden Extrapolieren bestimmt werden. Die Extrapolationseinrichtung 5 kann die Extrapolationsfunktion basierend auf der Änderung zumindest eines Betriebszustandswertes der Laservorrichtung bestimmen, wodurch eine genauere Bestimmung, d.h. Voraussage, des Austauschzeitpunktes eines Bauteils einer Laservorrichtung getroffen werden kann. Die Extrapolationseinrichtung 5 kann die Extrapolationsfunktion basierend auf der Änderung zumindest eines Betriebszustandswertes einer Mehrzahl von Laservorrichtungen bestimmen, wodurch eine Extrapolationsfunktion gewonnen wird, die von einer Exemplarstreuung unabhängig ist.

Bei einer weiteren Ausführungsform sendet die erste Laservorrichtung 12 und die zweite Laservorrichtung 22 die Betriebszustandswerte mit einer Kommunikationseinrichtung 19 an eine Sammeleinrichtung 18. Die Sammeleinrichtung 18 kann Betriebszustandswerte einer Mehrzahl von Laservorrichtungen vom gleichen und/oder einem anderen Typ sammeln. Vorzugsweise ist die Sammeleinrichtung 18 dem gleichen ophthalmologischen Behandlungsbereich wie die entsprechenden Laservorrichtungen zugeordnet und/oder befindet sich dort. Die Sammeleinrichtung 18 kann auch Betriebszustandswerte eines mechanischen Mikrokeratoms (nicht gezeigt) sammeln. Vorzugsweise kommuniziert die Sammeleinrichtung 18 über ihre Kommunikationseinrichtung 19, die eine Mehrzahl unterschiedlicher Kommunikationsschnittstellen bereitstellen kann, mit einer Mehrzahl von ophthalmologischen Vorrichtungen unterschiedlichen Typs, beispielsweise einer Laservorrichtung, einem mechanischen Mikrokeratom oder dergleichen. Folglich ist es nicht erforderlich, dass jede ophthalmologische Vorrichtung eine Kommunikationseinrichtung umfasst und ein entsprechendes Kommunikationsverfahren ausführen kann, die bzw. das dazu geeignet sind, mit der Überwachungseinrichtung 2 zu kommunizieren, da lediglich die Sammeleinrichtung 18 eine Kommunikationseinrichtung 19 umfassen muss, die dazu geeignet sind, mit der Überwachungseinrichtung 2 zu kommunizieren. Es versteht sich, dass die Sammeleinrichtung 18 eine Kommunikationseinrichtung 19 umfassen kann, die mehrere Kommunikationsschnittstellen bereitstellt, oder auch eine Mehrzahl von Kommunikationseinrichtungen umfassen kann, die jeweils eine Kommunikationsschnittelle bereitstellen.

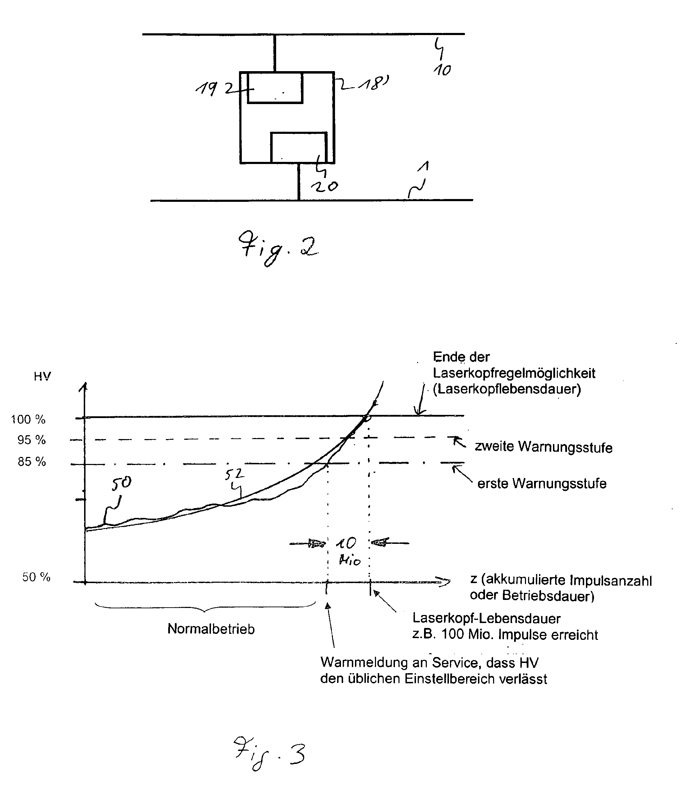

Fig. 2 zeigt eine weitere, mit dem Bezugszeichen 18' gekennzeichnete Ausführungsform der Sammeleinrichtung, die eine Kommunikations-Schnittstelle 19 umfasst, um über ein lokales Netzwerk 10 mit den ophthalmologischen Vorrichtungen 12, 22, zu kommunizieren. Die Sammeleinrichtung 18' umfasst ferner eine weitere Kommunikationseinrichtung 20, um mit einem weiteren Netzwerk, beispielsweise einem WAN (Wide Area Network) oder dem Internet 1, zu kommunizieren. Die Sammeleinrichtung 18' trennt somit das lokale Netzwerk 10 mit der ersten und zweiten ophthalmomolgischen Vorrichtung 12, 22 von dem weiteren Netzwerk, beispielsweise dem Internet 1, und führt somit zusätzlich die Funktionalität eines Netzwerkelements, beispielsweise einer Vermittlungseinrichtung, eines Routers und/oder einer Firewall, aus. Das lokale Netzwerk 10 kann auch als Busstruktur, beispielsweise CAN-Bus (Controller Area Network), ausgebildet sein.

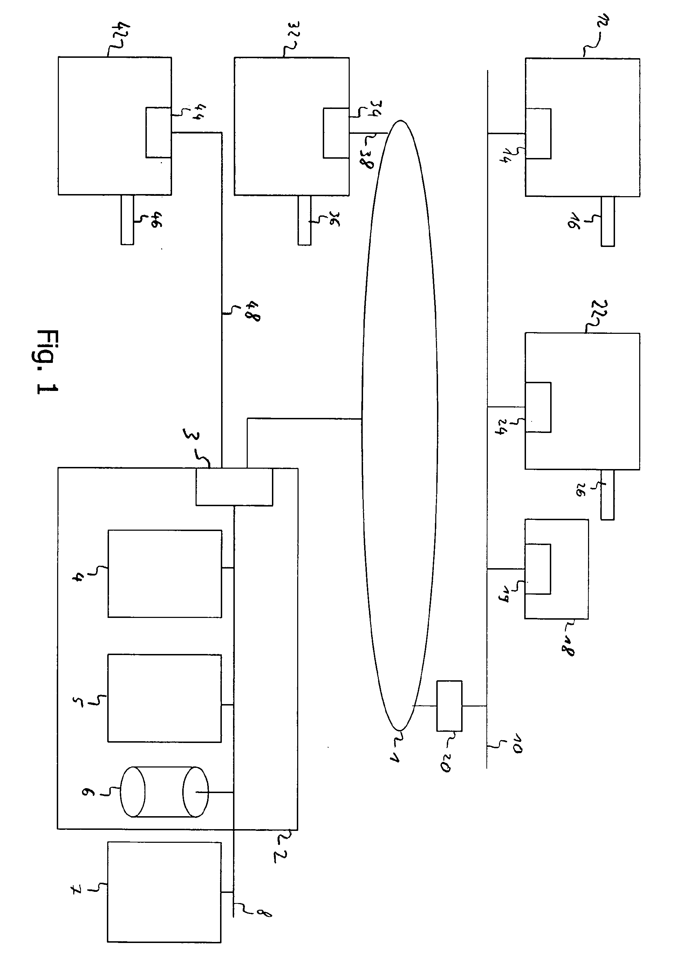

Es wird wieder auf Fig. 1 Bezug genommen. Bei einer weiteren Ausführungsform ermittelt die Auswerteeinrichtung 4 einen Vorgabewert basierend auf dem zumindest einen Betriebszustandswert, der an eine der Laservorrichtungen 12, 22, 32, 42 über eine der zuvor beschriebenen Kommunikationsverbindungen gesendet wird. Beispielsweise kann basierend auf der gemessenen Intensität des von einer Laservorrichtung abgegebenen Laserstrahles ein Betriebs-Vorgabewert der entsprechenden Laservorrichtung geändert werden. Aus Sicherheitsgründen kann dieser Vorgabewert durch einen Anwender oder einen Servicetechniker an der entsprechenden Laservorrichtung bestätigt werden, bevor er aktiviert wird.

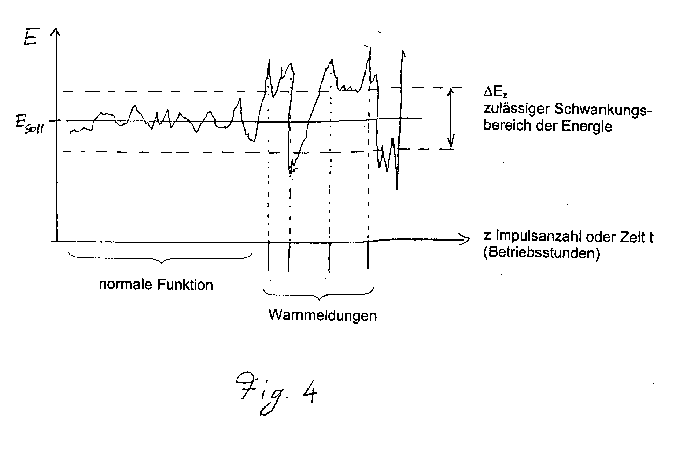

Fig. 3 zeigt die Hochspannungseinstellung (HV) für einen Excimer-Laserkopf eines refraktiven Lasersystems, die erforderlich ist, um die notwendige Soll-Energie eines ArF-Excimer-Lasers zu erreichen, beispielsweise eine Impulsenergie von etwa 4 mJ. Bezugszeichen 52 bezeichnet eine Kurve, die die Extrapolationsfunktion der zum Erzeugen eines Impulses mit einer bestimmten Energie erforderliche Hochspannungseinstellung über die Impulsanzahl oder die Betriebsdauer zeigt. Die Extrapolationsfunktion 52 kann mit dem erfindungsgemäßen Verfahren und/oder der erfindungsgemäßen Überwachungseinrichtung bestimmt werden. Mit dem erfindungsgemäßen Verfahren und/oder mit der erfindungsgemäßen Überwachungseinrichtung können bei einer Ausführungsform eine erste Warnungsstufe und eine zweite Warnungsstufe bestimmt werden. Die erste Warnungsstufe bezeichnet den Punkt, an dem noch etwa 10 Millionen Impulse mit dem Laserkopf erzeugt werden können. Bei der in Fig. 3 dargestellten Ausführungsform entspricht das etwa dem Punkt, an dem 85% der maximal zulässigen Hochspannung zum Erzeugen eines Impulses mit ausreichender Leistung eingestellt werden müssen. Wird die erste Warnungsstufe erreicht, kann automatisch ein Wartungseinsatz geplant werden. Die zweite Warnungsstufe wird erreicht, falls zum Erzeugen eines Laserimpulses mit ausreichender Leistung 95% der maximal zulässigen Hochspannung eingestellt werden müssen. Es kann eine zusätzliche Warnung an den Anwender der ophthalmologischen Einrichtung und/oder an einen Kundendienst ausgegeben werden, dass der Laserkopf demnächst ausgetauscht werden muss.

Fig. 4 zeigt eine Fernüberwachung der Impulsenergieschwankung ΔE_{z} eines Excimer-Lasersystems. Verlässt die Ist-Impulsenergie den zulässigen Schwankungsbereich, werden Warnmeldungen erzeugt, die von der ophthalmologischen Vorrichtung an die entfernte Überwachungseinrichtung gesendet werden können. Aufgrund der auftretenden Warnungen kann eine Wartung, beispielsweise automatisch, geplant werden. Ferner kann aufgrund der erzeugten Warnmeldung, d.h. des Betriebszustandswertes, die weitere Lebensdauer eines Bauteiles der ophthalmologischen Vorrichtung und eine potentielle Fehlerursache automatisch bestimmt werden. Bei einer Ausführungsform vergleicht das Verfahren zumindest eine von einer ophthalmologischen Vorrichtung erzeugte Warnmeldung, d.h. einen Betriebszustandswert, mit einer Warnmeldung, d.h. einem Betriebszustandswert, von einer anderen ophthalmologischen Vorrichtung.

Die vorliegende Erfindung hat den Vorteil, dass sie den Wartungszeitpunkt für zumindest ein Bauteil einer ophthalmologischen Vorrichtung relativ genau bestimmt. Durch diese relativ genaue Bestimmung werden der Behandlungserfolg und die Sicherheit eines Patienten gewährleistet. Ferner wird ein Bauteil einer ophthalmologischen Laservorrichtung nicht vorzeitig ausgetauscht, was den Aufwand zum Betrieb der ophthalmologischen Laservorrichtung reduziert. Ferner können durch die vorliegende Erfindung effiziente Wartungsstrategien entwickelt werden, die einen guten Behandlungserfolg und eine hohe Sicherheit des Patienten sowie niedrigere Kosten gewährleisten. Die Überwachungseinrichtung kann eine Mehrzahl von ophthalmologischen Vorrichtungen aus der Ferne überwachen. Unter Umständen ist nur eine zentrale Überwachungseinrichtung erforderlich, um eine Mehrzahl von ophthalmologischen Vorrichtungen zu überwachen, die sich an beliebigen Orten auf der Welt befinden.

## Patentansprüche

1. Verfahren zum Bestimmen eines Wartungszeitpunktes für zumindest ein Bauteil zumindest einer ophthalmologischen Vorrichtung (12, 22, 32, 42), umfassend die folgenden Schritte:
Ermitteln zumindest eines Betriebszustandswertes, der auf den Betriebszustand zumindest eines Bauteils der ophthalmologischen Vorrichtung (12, 22, 32, 42) hinweist; Übertragen des zumindest einen Betriebszustandswertes von der ophthalmologischen Vorrichtung (12, 22, 32, 42) an eine entfernte Überwachungseinrichtung (2); und
Bestimmen des Wartungszeitpunktes des zumindest einen Bauteils der zumindest einen ophthalmologischen Vorrichtung basierend auf dem zumindest einen Betriebszustandswert der ophthalmologischen Vorrichtung (12, 22, 32, 42) in der entfernten Überwachungseinrichtung (2).

2. Verfahren nach Anspruch 1, ferner umfassend die folgenden Schritte:
Ermitteln des zumindest eines Betriebszustandswertes bei einer Mehrzahl von ophthalmologischen Vorrichtungen;
Übertragen des zumindest einen Betriebszustandswertes von der Mehrzahl von ophthalmologischen Vorrichtungen (12, 22, 32, 42) an die entfernte Überwachungseinrichtung (2); und
Bestimmen des Wartungszeitpunktes des zumindest einen Bauteils zumindest einer der Mehrzahl von ophthalmologischen Vorrichtungen basierend auf dem zumindest einen Betriebszustandswert der Mehrzahl von ophthalmologischen Vorrichtungen (12, 22, 32, 42) in der entfernten Überwachungseinrichtung (2).

3. Verfahren nach einem der vorstehenden Ansprüche,
**dadurch gekennzeichnet, dass** der Schritt des Bestimmens des Wartungszeitpunktes des zumindest einen Bauteils der zumindest einen ophthalmologischen Vorrichtung (12, 22, 32, 42) basierend auf einer Mehrzahl von Betriebszustandswerten der zumindest einen ophthalmologischen Vorrichtung (12, 22, 32, 42) durchgeführt wird.

4. Verfahren nach einem der vorstehenden Ansprüche,
**dadurch gekennzeichnet, dass** der Schritt des Bestimmens des Wartungszeitpunktes des zumindest einen Bauteils der zumindest einen ophthalmologischen Vorrichtung (12, 22, 32, 42) basierend auf einer Änderung des zumindest einen Betriebszustandswertes durchgeführt wird.

5. Verfahren nach Anspruch 4,
**dadurch gekennzeichnet, dass** der Schritt des Bestimmens des Wartungszeitpunktes des zumindest einen Bauteils der zumindest einen ophthalmologischen Vorrichtung (12, 22, 32, 42) basierend auf einer zeitlichen Änderung des zumindest einen Betriebszustandswertes durchgeführt wird.

6. Verfahren nach Anspruch 4 oder 5,
**dadurch gekennzeichnet, dass** der Schritt des Bestimmens des Wartungszeitpunktes des zumindest einen Bauteils der zumindest einen ophthalmologischen Vorrichtung (12, 22, 32, 42) basierend auf einer Änderung des zumindest einen Betriebszustandswertes in Abhängigkeit von der Einsatzhäufigkeit der ophthalmologischen Vorrichtung (12, 22, 32, 42) durchgeführt wird.

7. Verfahren nach einem der Ansprüche 4 bis 6,
**dadurch gekennzeichnet, dass** der Schritt des Bestimmens des Wartungszeitpunktes des zumindest einen Bauteils der zumindest einen ophthalmologischen Vorrichtung (12, 22, 32, 42) unter Verwendung einer Extrapolationsfunktion durchgeführt wird.

8. Verfahren nach einem der Ansprüche 4 bis 7,
**gekennzeichnet durch** den Schritt des Bestimmens der Extrapolationsfunktion basierend auf der Änderung des zumindest einen Betriebszustandeswertes einer ophthalmologischen Vorrichtung (12, 22, 32, 42).

9. Verfahren nach einem der Ansprüche 4 bis 7,
**gekennzeichnet durch** den Schritt des Bestimmens der Extrapolationsfunktion basierend auf der Änderung des zumindest einen Betriebszustandswertes einer Mehrzahl von ophthalmologischen Vorrichtungen (12, 22, 32, 42).

10. Verfahren nach einem der Ansprüche 2 bis 9,
**dadurch gekennzeichnet, dass** der Schritt des Bestimmens des Wartungszeitpunktes des zumindest einen Bauteils zumindest einer der Mehrzahl von ophthalmologischen Vorrichtungen (12, 22, 32, 42) unter Verwendung eines statistischen Verfahrens durchgeführt wird.

11. Verfahren nach einem der vorstehenden Ansprüche,
**dadurch gekennzeichnet, dass** der Schritt des Übertragens des zumindest einen Betriebszustandswertes ein Senden des zumindest einen Betriebszustandswertes an die entfernte Überwachungseinrichtung (2) umfasst.

12. Verfahren nach Anspruch 11,
**dadurch gekennzeichnet, dass** das Senden aufgrund eines vorbestimmen Ereignisses in zumindest einer ophthalmologischen Vorrichtung (12, 22, 32, 42) und/oder durch manuelle Auslösung erfolgt.

13. Verfahren nach einem der vorstehenden Ansprüche,
**dadurch gekennzeichnet, dass** es ferner die folgenden Schritte umfasst:
Übertragen des zumindest einen Betriebszustandswertes an eine Sammeleinrichtung (18), die dazu ausgebildet ist, zumindest einen Betriebszustandswert einer Mehrzahl von ophthalmologischen Vorrichtung (12, 22) zu empfangen, und
Senden des zumindest einen Betriebszustandswertes von der Sammeleinrichtung (18) an die entfernte Überwachungseinrichtung (2).

14. Verfahren nach Anspruch 13,
**dadurch gekennzeichnet, dass** der Schritt des Empfangens des zumindest einen Betriebszustandswertes an der Sammeleinrichtung (18) das Empfangen zumindest eines Betriebszustandswertes von einer Mehrzahl von ophthalmologischen Vorrichtungen (12, 22) unterschiedlichen Typs umfasst.

15. Verfahren nach einem der vorstehenden Ansprüche,
**gekennzeichnet durch** die Schritte des Bestimmen zumindest eines Vorgabewertes aus dem zumindest einen Betriebszustandswert in der entfernten Überwachungseinrichtung (2) und des Sendens des Vorgabewertes an zumindest eine ophthalmologische Vorrichtung (12, 22, 32, 42).

16. Überwachungseinrichtung (2), die dazu ausgelegt ist,
mit zumindest einer ophthalmologischen Vorrichtung (12, 22, 32, 42) zusammenzuwirken, die dazu ausgebildet ist, zumindest einen Betriebszustandswert zumindest eines Bauteils zu ermitteln und zu senden, umfassend:
eine Empfangseinrichtung (3), die dazu ausgebildet ist, den zumindest einen Betriebszustandswert von zumindest einer ophthalmologischen Vorrichtung (12, 22, 32, 42) zu empfangen, und
eine Auswerteeinrichtung (4), die dazu ausgebildet ist, einen Wartungszeitpunkt für zumindest ein Bauteil der zumindest einen ophthalmologischen Vorrichtung (12, 22, 32, 42) basierend auf dem zumindest einen Betriebszustandswert der zumindest einen ophthalmologischen Vorrichtung (12, 22, 32, 42) zu bestimmen.

17. Überwachungseinrichtung (2) nach Anspruch 16,
**dadurch gekennzeichnet, dass** die Auswerteeinrichtung (4) dazu ausgebildet ist, den Wartungszeitpunkt für zumindest ein Bauteil der zumindest einen ophthalmologischen Vorrichtung (12, 22, 32, 42) basierend auf dem zumindest einen Betriebszustandswert einer Mehrzahl von ophthalmologischen Vorrichtungen (12, 22, 32, 42) zu bestimmen.

18. Überwachungseinrichtung (2) nach Anspruch 16 oder 17,
**dadurch gekennzeichnet, dass** die Auswerteeinrichtung (4) dazu ausgebildet ist, den Wartungszeitpunkt des zumindest einen Bauteils der zumindest einen ophthalmologischen Vorrichtung (12, 22, 32, 42) basierend auf einer Mehrzahl von Betriebszustandswerten der zumindest einen ophthalmologischen Vorrichtung (12, 22, 32, 42) zu bestimmen.

19. Überwachungseinrichtung (2) nach einem der Ansprüche 16 bis 18,
**dadurch gekennzeichnet, dass** die Auswerteeinrichtung (4) dazu ausgebildet ist, den Wartungszeitpunkt des zumindest einen Bauteils der zumindest einen ophthalmologischen Vorrichtung (12, 22, 32, 42) basierend auf einer Änderung des zumindest einen Betriebszustandswertes zu bestimmen.

20. Überwachungseinrichtung (2) nach Anspruch 19,
**dadurch gekennzeichnet, dass** die Auswerteeinrichtung (4) dazu ausgebildet ist, den Wartungszeitpunkt des zumindest einen Bauteils der zumindest einen ophthalmologischen Vorrichtung (12, 22, 32, 42) basierend auf einer zeitlichen Änderung des zumindest einen Betriebszustandswertes zu bestimmen.

21. Überwachungseinrichtung (2) nach Anspruch 19 oder 20,
**dadurch gekennzeichnet, dass** die Auswerteeinrichtung (4) dazu ausgebildet ist, den Wartungszeitpunkt des zumindest einen Bauteils der zumindest einen ophthalmologischen Vorrichtung (12, 22, 32, 42) basierend auf einer Änderung des zumindest einen Betriebszustandswertes in Abhängigkeit von der Einsatzhäufigkeit der ophthalmologischen Vorrichtung (12, 22, 32, 42) zu bestimmen.

22. Überwachungseinrichtung (2) nach einem der Ansprüche 19 bis 21, **gekennzeichnet durch** eine Extrapolationseinrichtung (5), die dazu ausgebildet ist, den Wartungszeitpunkt des zumindest einen Bauteils der ophthalmologischen Vorrichtung (12, 22, 32, 42) unter Verwendung einer Extrapolationsfunktion zu bestimmen.

23. Überwachungseinrichtung (2) nach Anspruch 22,
**dadurch gekennzeichnet, dass** die Extrapolationseinrichtung (5) dazu ausgebildet ist, die Extrapolationsfunktion basierend auf der Änderung des zumindest einen Betriebszustandswertes einer ophthalmologischen Vorrichtung (12, 22, 32, 42) zu bestimmen.

24. Überwachungseinrichtung (2) nach Anspruch 22 oder 23,
**dadurch gekennzeichnet, dass** die Extrapolationseinrichtung (5) dazu ausgebildet ist, die Extrapolationsfunktion basierend auf der Änderung des zumindest einen Betriebszustandswertes einer Mehrzahl von ophthalmologischen Vorrichtungen (12, 22, 32, 42) zu bestimmen.

25. Überwachungseinrichtung (2) nach einem der Ansprüche 17 bis 24,
**dadurch gekennzeichnet, dass** die Auswerteeinrichtung (4) dazu ausgebildet ist, den Wartungszeitpunkt des zumindest einen Bauteils der zumindest einen ophthalmologischen Vorrichtung (12, 22, 32, 42) unter Verwendung eines statistischen Verfahrens zu bestimmen.

26. Überwachungseinrichtung (2) nach einem der Ansprüche 16 bis 25,
**dadurch gekennzeichnet, dass** die Auswerteeinrichtung (4) dazu ausgebildet ist, zumindest einen Vorgabewert aus dem zumindest einen Betriebszustandswert zu bestimmen.

27. Überwachungssystem umfassend,
eine Überwachungseinrichtung (2) nach einem der Ansprüche 16 bis 26 und eine Sammeleinrichtung (18; 18'), die dazu ausgebildet ist, zumindest einen Betriebszustandswert einer Mehrzahl von ophthalmologischen Vorrichtungen (12, 22, 32, 42) zu empfangen und den zumindest einen Betriebszustandswert an die Überwachungseinrichtung (2) zu senden.

28. Überwachungssystem nach Anspruch 27,
**dadurch gekennzeichnet, dass** die Sammeleinrichtung (18, 18') dazu ausgebildet ist, zumindest einen Betriebszustandswert einer Mehrzahl von ophthalmologischen Vorrichtungen (12, 22, 32, 42) unterschiedlichen Typs zu empfangen, zu speichern und an die Überwachungseinrichtung (2) zu senden.

29. Überwachungssystem nach einem der Ansprüche 27 oder 28, **dadurch gekennzeichnet, dass** die Sammeleinrichtung (18') dazu ausgebildet ist, ein erstes Netzwerk (10) von einem zweiten Netzwerk (1) zu trennen.

30. Computerprogrammprodukt mit einem auf einem maschinenlesbaren Träger gespeicherten Programmcode zur Durchführung des Verfahrens nach einem der Ansprüche 1 bis 15, wenn der Programmcode in einem Computer geladen ist oder in einem Computer ausgeführt wird.
